# EUROPEAN PATENT SPECIFICATION

(11) **EP 2 411 807 B1**
(45) Date of publication and mention of the grant of the patent: **20.08.2014**
(21) Application number: 10756511.1
(22) Date of filing: 07.01.2010
(51) Int. Cl.: G01N 33/53, C12Q 1/68, G01N 33/68, C12N 15/10, C12N 15/115

(54) **Methods for simultaneous generation of functional aptamers**
Verfahren zur simultanen Erzeugung funktioneller Aptameren
Procédés de génération simultanée d'aptamères fonctionnels

(30) Priority: 23.03.2009 US 162394 P; 06.06.2009 US 479806
(43) Date of publication of application: 01.02.2012
(73) Proprietor: Base Pair Biotechnologies, Inc., Houston, TX 77054 (US)
(72) Inventor: JACKSON, George, Houston, TX 77054 (US)
(74) Representative: Carpintero Lopez, Francisco
(86) International application number: PCT/US2010/020287
(87) International publication number: WO 2010/110929

(56) References cited:
- WO-A1-02/083951
- WO-A2-02/099078
- US-A- 6 087 103
- US-A1- 2003 148 335
- US-A1- 2003 148 335
- US-A1- 2003 228 619
- US-A1- 2004 121 331
- US-A1- 2007 166 741
- MORRIS, K. N. ET AL.: "High Affinity Ligands from in vitro Selection: Complex Targets", PNAS, vol. 95, no. 6, March 1998 (1998-03), pages 2902-2907, XP002207684,
- LAYZER J M ET AL: "Simultaneous Generation of Aptamers to Multiple Gamma-Carboxyglutamic Acid Proteins From a Focused Aptamer Library Using DeSELEX and Convergent Selection", OLIGONUCLEOTIDES, MARY ANN LIEBERT, NEW YORK, NY, US, vol. 17, no. 1, 26 April 2007 (2007-04-26) , pages 1-11, XP002560970, ISSN: 1545-4576, DOI: 10.1089/OLI.2006.0059
- MORRIS, K. N. ET AL.: 'High Affinity Ligands from in vitro Selection: Complex Targets' PNAS vol. 95, no. 6, March 1998, pages 2902 - 2907, XP002207684
- KNIGHT, C. G. ET AL.: 'Array-Based Evolution of DNA Aptamers Allows Modelling of an Explicit Sequence-Fitness Landscape' NUCLEIC ACID RES. vol. 37, no. 1, November 2008, XP008155206
- NOMA, T. ET AL.: 'Screening of DNA Aptamers against Multiple Proteins in Tissue' NUCLEIC ACIDS SYMP. SER. vol. 49, 2005, pages 357 - 359, XP008155220

## Description

### SEQUENCE LISTING

Deoxyribonucleic acid sequences are hereby incorporated by reference to the ASCII text file entitled "P1011US01_ST25.txt", created January 6, 2010, of 9.11 kilobytes in size.

### FIELD OF THE INVENTION

This invention relates to methods for generating multiple functional aptamers against multiple different target molecules simultaneously.

### BACKGROUND OF THE INVENTION

Aptamers, which are nucleic acid ligands capable of binding to molecular targets, have recently attracted increased attention for their potential application in many areas of biology and biotechnology. They may be used as sensors, therapeutic tools, to regulate cellular processes, as well as to guide drugs to their specific cellular target(s). Contrary to the actual genetic material, their specificity and characteristics are not directly determined by their primary sequence, but instead by their tertiary structure. Aptamers have been recently investigated as immobilized capture elements in a microarray format. Others have recently selected aptamers against whole cells and complex biological mixtures.

Aptamers are commonly identified by an in vitro method of selection sometimes referred to as Systematic Evolution of Ligands by EXponential enrichment or "SELEX". SELEX typically begins with a very large pool of randomized polynucleotides which is generally narrowed to one aptamer ligand per molecular target. Once multiple rounds (typically 10-15) of SELEX are completed, the nucleic acid sequences are identified by conventional cloning and sequencing. Aptamers have most famously been developed as ligands to important proteins, rivaling antibodies in both affinity and specificity, and the first aptamer-based therapeutics are now emerging. More recently, however, aptamers have been also developed to bind small organic molecules and cellular toxins, viruses, and even targets as small as heavy metal ions.

US 2003148335 A1 discloses a method for assaying a plurality of different non-nucleic acid targets in a sample. Aptamers are mentioned as possible ligands (see for example paragraph [0101]) or target-binding portion of the reporter ligands (see for example paragraph [0016]), thus, they are already identified as binding to the particular targets assayed. The ligands are tagged before they bind to the targets, so the tags do not serve to associate the ligands with the targets they bind to, but rather are only used to identify which ligand is present.

LAYZER J M et al. "Simultaneous Generation of Aptamers to Multiple Gamma-Carboxyglutamic Acid Proteins From a Focused Aptamer Library Using DeSELEX and Convergent Selection", (OLIGONUCLEOTIDES, MARY ANN LIEBERT, NEW YORK, NY, US, vol. 17, no. 1, 26 April 2007 (2007-04-26), pages 1-11), discloses a method for simultaneous generation of aptamers against a complex proteome of multiple gamma-carboxyglutamic acid proteins using DeSELEX and convergent selection to direct selection of aptamers against multiple targets present in very different concentrations in a mixture. This method deselects aptamers that bind to the dominant protein in the mixture so the selection can be redirected to select for the less abundant proteins.

### SUMMARY OF THE INVENTION

The present invention is defined by the appended claims. Thus, the invention refers to:
1. A method for simultaneously selecting a plurality of different aptamers for multiple targets comprising the following steps:
   a) contacting a library of aptamers with a plurality of targets simultaneously, wherein the plurality of targets are affixed to a substrate within a single volume and disposed in the form of an array of spots with each target identifiable by the location of a particular spot on the substrate, wherein each member of the library includes: a potential target-binding sequence, at least one conserved region capable of binding to an identifier oligonucleotide by hybridization, and a further conserved region for priming nucleic acid amplification reactions;
   b) partitioning members of said library that do not bind to said plurality of targets;
   c) marking the members of the library that bind target spots with identifier oligonucleotides, where each identifier oligonucleotide includes: a unique sequence that is utilized to correspond to the location of a target on the array, a conserved region capable of binding to the conserved region of the library members by hybridisation, and a further conserved region for priming nucleic acid amplification reactions; and
   d) identifying the binding members of the library and correlating them to the targets via the unique sequence of the identifier utilizing nucleic acid amplification to generate copies of the binding members with the unique sequence of the identifier oligonucleotide incorporated into the amplified product.
2. The method of claim 1, wherein the single reaction volume includes multiple reaction sub-volumes.
3. The method of claim 1 or 2 wherein the plurality of targets are disposed on a substrate with multiple copies of at least one target.
4. The method of claim 1, 2 or 3, wherein the partitioning comprises washing.
5. The method of claim 4 further comprising repeating the contacting and washing steps.
6. The method of any of claims 1-5, wherein the contacting step comprises manual pipetting, automatic pipetting, inkjet printing, applying micro-contact pins or applying a membrane with identifiers disposed thereon.
7. The method of any of claim 1-6, further comprising attaching, ligating, photoligating, or covalently bonding the identifiers to the aptamers.
8. The method of any of claims 1-7 further comprising performing at least one round of a SELEX protocol with the library of aptamers against multiple targets prior to or after contacting with the plurality of targets on the substrate.
9. A system for performing the method of claim 1 comprising:
   a) the library as specified in claim 1;
   b) the plurality of targets as specified in claim 1; and
   c) the plurality of identifiers as specified in claim 1.
10. The system of claim 9, wherein the array of spots disposed on the substrate has a spatial arrangement
11. The system of claim 9 or 10, wherein the identifiers comprise nucleic acids having unique or semi-unique sequences that correspond to the plurality of targets.
12. The system of claim 9, 10 or 11, wherein the identifiers comprise primers for the bound aptamers.
13. The system of claim 9, 10, 11 or 12, wherein the identifiers are synthesized in situ on the substrate.
14. The system of claim 9, 10, 11, 12 or 13, wherein the identifiers are disposed on a separate substrate matching the same spatial arrangement as the disposition of targets on the substrate.

### OTHER ASPECTS OF THE DISCLOSURE

The present disclosure more generally relates to methods for simultaneously generating, for example, numerous different functional biomolecules, particularly to methods for generating numerous different functional nucleic acids against multiple target molecules simultaneously. The present disclosure further relates to methods for generating, for example, functional biomolecules, particularly to functional nucleic acids, that bind with functional activity to another biomolecule, such as a receptor molecule. In the present invention, the biomolecules are, aptamers. More than one or multiple targets as used herein may generally include different types of targets, and/or may also include a multitude of a singular type of targets at different conditions, such as, for example, temperature, pH, chemical environment, and/or any other appropriate conditions.

In general, a method for generating functional biomolecules includes obtaining a library, such as a diverse or randomized library, for example, of biomolecules. A library of biomolecules may include nucleic acid sequences, such as ribonucleic acid (RNA), deoxyribonucleic acid (DNA), artificially modified nucleic acids, and/or combinations thereof. Again, the biomolecules are aptamers in the present invention. The method for generating functional biomolecules further includes contacting the library of biomolecules with more than one target, such as, for example, a molecular target, material and/or substance. In general, the members of the library that do not bind with some affinity to the more than one target may be washed or otherwise partitioned from the remainder of the library, which may have a given level of binding affinity to the more than one target. The process may be repeated to partition the strongest binding members of the library. Amplification of the biomolecules may also be utilized to increase the numbers of the binding members of the library for subsequent repetitions and for isolation and/or purification of any final products of the process. Embodiments of the SELEX method may generally be utilized to achieve the generation of functional biomolecules of a given binding affinity, such biomolecules generally referred to as aptamers or ligands.

In one exemplary aspect of the invention, generation of functional biomolecules may be performed against more than one or multiple targets simultaneously within a single system, such as the generation of functional nucleic acid ligands within a single reaction volume. In general, more than one or a plurality of targets may be disposed within in a single reaction volume, and a library of biomolecules, such as a nucleic acid library, may be applied to the reaction volume. The members of the library that do not bind to any of the plurality of targets under given conditions may then be partitioned, such as by washing. One or more rounds of binding and partitioning of the members of the library may be performed, such as, for example, to obtain a remainder of members of the library with a given affinity for their targets. The remaining members that bind to the plurality of targets of the library may then be marked and/or tagged, such as to identify the particular target or targets to which the member(s) of the library binds. The binding members of the library may then be isolated and, by virtue of the marking or tagging, be matched to a particular target or targets. This is desirable as high capacity, multiplexed identification procedures may save time, expense, and physical space for the process over single target identification processes. The present method may also be desirable as it may be utilized to identify and/or eliminate biomolecules that bind or have a tendency to bind to multiple targets.

In an exemplary embodiment, a plurality of target molecules are affixed to a substrate within a single reaction volume, such as, for example, by attaching the targets to a substrate of an array. It may generally be appreciated that a single reaction volume may refer to or include multiple reaction sub-volumes, such as, for example, discrete or semi-discrete fluid droplets. In general, the targets may be disposed with multiple copies of each target in clusters or "spots" such that a given array may have an ordered deposition of targets on the substrate, with each target identifiable by the location of a particular spot on the substrate. A library of nucleic acids may then be contacted or applied to the array and the non-binding members of the library may be partitioned or washed off the array. The binding and washing steps may be repeated and may also utilize an amplification step to generate additional copies of any remaining binding members of the library. The array may then be marked or tagged with a plurality of identifiers, such as, for example, a plurality of oligonucleotides which may universally bind through Watson-Crick interactions to the members of the library of nucleic acids. The marking or tagging may be, for example, accomplished by manually applying identifiers, such as by pipetting or the like, utilizing microcontact pins, applying membranes/films with identifiers, printing, for example, inkjet printing, and/or other similar tagging methods, of identifier containing solutions to the array. The identifiers may further include a unique or semi-unique sequence which may be utilized to correspond to the spots and thus the targets of the array. For example, a unique or semi-unique identifier sequence may be utilized that identifies each spatial location on an array, such as each particular target spot. The identifier may then be associated with and/or attached to the nucleic acid members bound to a particular spot. Thus, the nucleic acids, for example, bound to a particular target spot may be identified by the sequence of the associated identifier. In some embodiments, the identifiers may further be primers and may be utilized with a nucleic acid amplification reaction on the array to generate additional copies of the bound nucleic acids. The unique or semi-unique identifier sequence may also be incorporated into the members of the library amplified. This may be desirable for associating a given member with a target or targets while preserving the particular sequence of the member as the locational identifying sequence is appended to the sequence of the library member. This may be particularly desirable for resolving multiple binders to a single target or members of the library that bind to multiple targets.

In general, the starting library of biomolecules, i.e. aptamers, may be the product of at least one round of a previous SELEX protocol. For example, at least one round of SELEX may be performed with a library of biomolecules against multiple targets, such as, for example, in a solution. The targets in the solution may be substantially identical to the targets disposed on an array. This may be desirable as multiple rounds of selection may be performed with a library prior to applying the remaining members to an array for marking/tagging. Complex target arrays may generally be more expensive and/or difficult to make or utilize than solutions of target molecules, so performing only the final binding and marking/tagging procedure on the array may be desirable.

In other embodiments, identifiers may be predisposed on the array substrate in substantial proximity to the spots such that they may bind to, for example, nucleic acids bound to the target spots. The identifiers may, for example, be covalently attached to the substrate. In some embodiments, the attachments may be controllably breakable or cleavable such that the identifiers may be released from the substrate such that they may, for example, more easily bind to the bound nucleic acids on the spots.

In further embodiments, identifiers may be synthesized in situ on the array, such as by light directed in situ nucleic acid synthesis. Appropriately sequenced identifiers may then be synthesized in proximity to particular spots such that the newly synthesized identifiers may bind to the nucleic acids bound to the target spot.

In still other embodiments, identifiers may be disposed and/or synthesized on a separate substrate, such as a membrane, in a spatial disposition that substantially matches the spatial disposition of spots on the array, i.e. the identifiers may be arranged such that they may be readily superimposed onto the target spots on the array. The identifier substrate may then be contacted with the array with locational matching of the spots with identifiers. The identifiers may then bind to the nucleic acids bound to the target spots. Any appropriate method of facilitating binding may be utilized, such as, for example, actions to drive migration of the identifiers to the array, such as capillary action, electrophoresis, pressure, gravitational settling, and/or any other appropriate method or combination thereof. The separate substrate may also be soluble, erodible, substantially permeable to the identifiers, and/or otherwise adapted for facilitating migration of the identifiers to the array.

In yet still other embodiments, the array substrate may be physically divided and/or partitioned for separate collection of the, for example, nucleic acids bound to the spots. The spots may, for example, also be controllably removable from the substrate such that they may be individually recovered and sorted.

In still yet other embodiments, identifiers may be disposed and/or synthesized on a separate substrate, such as a membrane, in a spatial disposition that substantially matches the spatial disposition of spots on the array, i.e. the identifiers may be arranged such that may be readily superimposed onto the target spots on the array. The separate substrate may be kept separately while the array substrate maybe physically divided and/or partitioned for separate collection of the nucleic acids bound to the spots. In this manner, the location of the different nucleic acids maybe maintained even when the array substrate is no longer intact, if the locations are of value. The identifiers may also be selectively applied to particular locations on the array and/or applied in a particular order or in groups.

In some embodiments, identifiers may only be applied to spots with bound nucleic acids. The spots with bound nucleic acids may be detected, for example, by detecting the presence of nucleic acids, such as by applying nucleic acid binding dyes, such as SYBR dyes, ethidium bromide and/or other appropriate dyes. The members of the nucleic acid library may also include detectable portions, such as, for example, fluorescent moieties, radioactive tags and/or other appropriate detectable portions.

In some embodiments, the identifiers may be applied to the bound nucleic acids together with other materials, such as for example, components of a nucleic acid amplification reaction, a nucleic acid ligation reaction, photo-linking reagents, and/or any other appropriate material, such as those materials that may facilitate attachment or association of the identifiers to the bound nucleic acids.

In yet another embodiment, identifiers may be ligated to the, for example, bound nucleic acids. For example, a nucleic acid ligase may be utilized to covalently link an identifier sequence to the bound nucleic acid. Further nucleic acid fragments may be utilized to facilitate ligase action, such as appropriate complementary fragments that may aid the formation of a substantially double-stranded nucleic acid complex compatible with a ligase. For another example, photo-ligation may be used to attach the identifiers to the, for example, bound nucleic acids. Photo-ligation may be especially useful when certain substrates are used. For example, macroporous substrates.

In general, methods may be applied that may facilitate binding or other interactions between the identifiers and the, for example, nucleic acids bound to the spots. For example, the temperature may be increased to dissociate the nucleic acids from the spots. The temperature may subsequently be lowered such that, for example, base pairing may occur between the nucleic acids and the identifiers. Further in general, it may be desirable to apply the identifiers in a manner that physically separates and/or isolates the individual target spots such that cross-marking due to identifier diffusion/migration may be minimized. For example, the identifiers may be applied in individual fluid droplets such that there is no continuous fluid contact between individual identifier containing fluids. For further example, the substrate of the array may be absorbent and/or porous such that the identifiers may be absorbed into the substrate material. The substrate material may also block lateral diffusion while allowing vertical diffusion, such that identifiers may be applied and absorbed into the substrate while minimizing diffusion across the plane of the substrate, such as to other target spots.

In a further aspect of the disclosure, a method for generating functional biomolecules includes obtaining a library of peptide sequences and contacting the library with a plurality of targets. In some exemplary aspects, the peptide sequence may be tagged, linked, marked and/or otherwise associated with a nucleic acid sequence. The nucleic acid sequence may be, for example, representative of the sequence of the peptide. For example, the nucleic acid may substantially encode the peptide sequence. Also for example, the nucleic acid may be a unique or semi-unique identifier sequence. The nucleic acid sequence may then be utilized to bind another identifier, as described above, such that a peptide bound to a target may be tagged or marked as to which target it bound.

In an exemplary aspect of the disclosure, a bacteriophage (phage) may be generated that includes a peptide sequence of interest in its protein coat. The phage may further include a nucleic acid sequence that may be representative of the peptide sequence within the nucleic acid of the phage. The phage may then be contacted with a plurality of targets, as above. This may generally be referred to as phage display. Non-binding phages may be washed and/or partitioned, while binding phages may be tagged or marked with identifiers, as above. As phage nucleic acids are generally contained within the protein coat of the phage, the nucleic acid may generally be exposed for binding to the identifier. For example, the phage may be heated such that the protein coat denatures and/or disassembles such that the nucleic acid is exposed. The identifier may also be introduced into the phage, such as by electroporation, electrophoresis, and/or any other appropriate method.

Other methods of peptide selection may include, but are not limited to, mRNA display, ribosome display, and/or any other appropriate peptide display method or combination thereof.

In another aspect of the disclosure, methods for handling and sorting the resultant sequences of a multiplexed binding process are provided. In some embodiments of the disclosure, the sequences may be sorted by identifier sequences to establish which target or targets the sequence bound. The sequences may further be compared, aligned and/or otherwise processed to identify features, characteristics and/or other useful properties, relationships to each other, and/or target properties.

In a further aspect of the disclosure, methods for monitoring and/or controlling the diversity of the library of biomolecules may be utilized. For example, too few rounds of selection may result in a biomolecule pool with too many weak binding members while too many rounds of selection may result in only a few binding members, such as members corresponding to only a few targets rather than members corresponding to all of the targets present. In one embodiment of the disclosure, Cot analysis may be employed to measure and/or monitor the diversity of the library of biomolecules through multiple rounds of selection. Cot, or Concentration x time, analysis measures the annealing time of particular oligonucleotides while in solution with other nucleic acids, such as the members of the library of biomolecules. In general, the annealing time will be faster the lower the diversity of the library.

### BRIEF DESCRIPTION OF THE FIGURES

FIG. 1 illustrates an embodiment of a multiple target array;
FIG. 2 illustrates the application of a library of biomolecules to a target array;
FIG. 2a illustrates the binding of members of a library of biomolecules to a target spot;
FIGs. 3 and 3a illustrate embodiments of biomolecule library members;
FIG. 3b illustrates an embodiment of an identifier;
FIG. 4 illustrates the tagging of a library member bound to target with an identifier;
FIG. 4a illustrates a tagged library member product;
FIG. 5 illustrates a target spot with nearby identifiers on a substrate;
FIG. 5a illustrates the application of an identifier sheet to a target array;
FIGs 6, 6a, 6b and 6c illustrate embodiments of identifiers and ligation of identifiers to a library member;
FIGs. 7 and 7a illustrate phage display for a target;
FIG. 7b illustrates an mRNA display fusion product;
FIG. 7c illustrates a ribosome display fusion product; and
FIG. 8 illustrates an example of a histology section target.

### DETAILED DESCRIPTION OF THE INVENTION

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the exemplified methods, devices and materials are now described.

The present invention is defined by the claims.

In general, a method for generating functional biomolecules includes obtaining a library, such as a diverse or randomized library, of biomolecules. In the present invention, biomolecules, which generally include nucleic acids, refer to aptamers. A library of biomolecules may include nucleic acid sequences, such as ribonucleic acid (RNA), deoxyribonucleic acid (DNA), artificially modified nucleic acids, and/or combinations thereof. In general, modified nucleic acid bases may be utilized and may include, but are not limited to, 2'-Deoxy-P-nucleoside-5'-Triphosphate, 2'-Deoxyinosine-5'-Triphosphate, 2'-Deoxypseudouridine-5'-Triphosphate, 2'-Deoxyuridine-5'-Triphosphate, 2'-Deoxyzebularine-5'-Triphosphate, 2-Amino-2'-deoxyadenosine-5'-Triphosphate, 2-Amino-6-chloropurine-2'-deoxyriboside-5'-Triphosphate, 2-Aminopurine-2'-deoxyribose-5'-Triphosphate, 2-Thio-2'-deoxycytidine-5'-Triphosphate, 2-Thiothymidine-5'-Triphosphate, 2'-Deoxy-L-adenosine-5'-Triphosphate, 2'-Deoxy-L-cytidine-5'-Triphosphate, 2'-Deoxy-L-guanosine-5'-Triphosphate, 2'-Deoxy-L-thymidine-5'-Triphosphate, 4-Thiothymidine-5'-Triphosphate, 5-Aminoallyl-2'-deoxycytidine-S'-Triphosphate, 5-Aminoallyl-2'-deoxyuridine-5'-Triphosphate, 5-Bromo-2'-deoxycytidine-5'-Triphosphate, 5-Bromo-2'-deoxyuridine-5'-Triphosphate, 5-Fluoro-2'-deoxyuridine-5'-Triphosphate, and/or any other appropriate modified nucleic acid base. It may generally be understood that the nucleoside triphosphates (NTPs) listed above may generally refer to any appropriate phosphate of the modified base, such as additionally, for example, monophosphates (NMPs) or diphosphates (NDPs) of the base. The method for generating functional biomolecules further includes contacting the library of biomolecules with at least one target, such as, for example, a molecular target, material and/or substance. In general, the members of the library that do not bind with some affinity to the target may be washed or otherwise partitioned from the remainder of the library, which may have a given level of binding affinity to the target. The process may be repeated to partition the strongest binding members of the library. Amplification of the biomolecules may also be utilized to increase the numbers of the binding members of the library for subsequent repetitions and for isolation and/or purification of any final products of the process. Embodiments of the SELEX method may generally be utilized to achieve the generation of functional biomolecules of a given binding affinity. The basic SELEX protocol is described in U.S. Patent No. 5,270,163, entitled "Methods for identifying nucleic acid ligands".

In one exemplary aspect of the invention, generation of functional biomolecules may be performed against multiple targets simultaneously within a single system, such as the generation of functional nucleic acid ligands within a single reaction volume. In general, a plurality of targets may be disposed within in a single reaction volume and a library of biomolecules, such as a nucleic acid library, may be applied to the reaction volume. The targets may be, for example, proteins, cells, small molecules, biomolecules, and/or combinations or portions thereof. The members of the library that do not bind to any of the plurality of targets under given conditions may then be partitioned, such as by washing. The remaining members of the library may then be marked and/or tagged, such as to identify the particular target or targets to which the member of the library binds. The binding members of the library may then be isolated and, by virtue of the marking or tagging, be matched to a particular target or targets. This may be desirable as high capacity, multiplexed identification procedures may save time, expense, and physical space for the process over single target identification processes. The present method may also be desirable as it may be utilized to identify and/or eliminate molecules that bind to multiple targets.

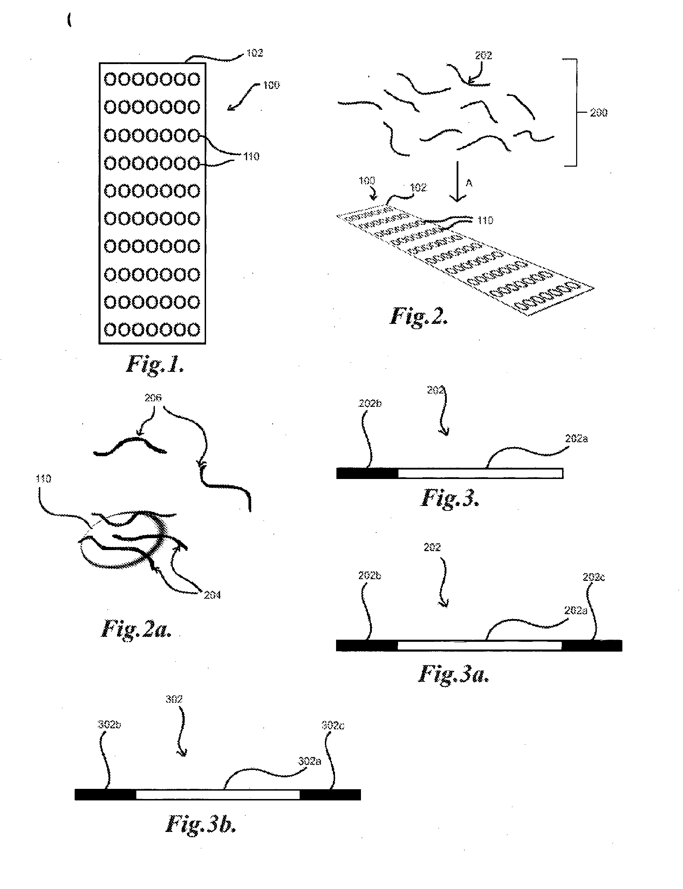

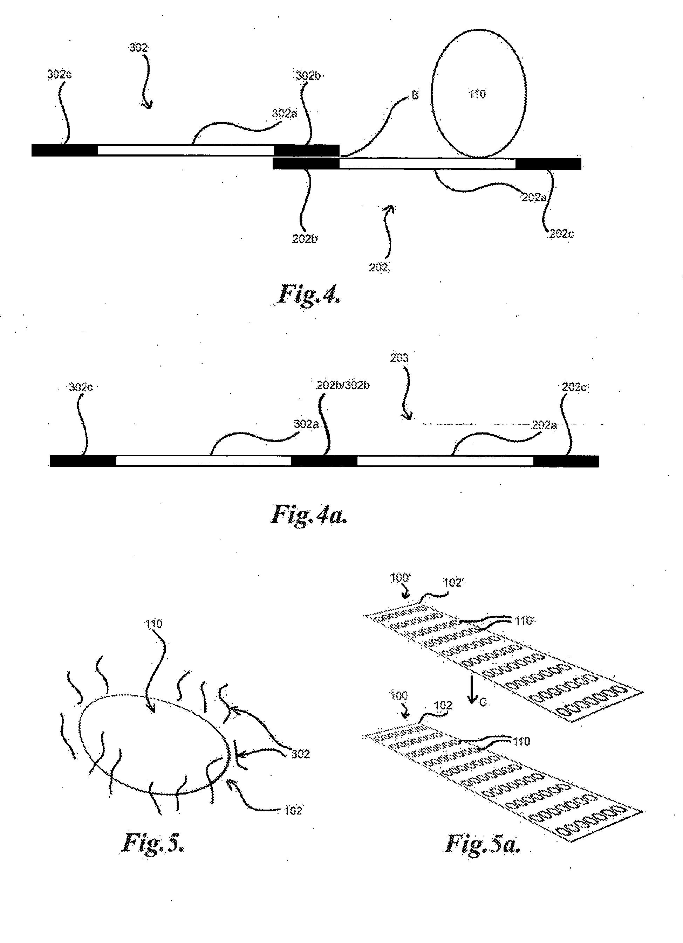

In an exemplary embodiment, a plurality of target molecules are affixed to a substrate within a single reaction volume, such as, for example, by attaching the targets to a substrate of an array. As illustrated in FIG. 1, the targets may be disposed with multiple copies of each target, such as target molecules, in clusters or "spots" 110 on the substrate 102 of an array 100 such that a given array 100 may have an ordered deposition of targets on the substrate 102, with each target identifiable by the location of a particular spot 110 on the substrate 102. Each spot 110 may be a unique target or there may be multiple spots 110 of at least one target on a given array 100. In general, high content target arrays, such as high content protein or antibody arrays, may be utilized. A library 200 of, for example, nucleic acids 202 may then be applied A to array 100, as illustrated in FIG. 2. Particular members 204 of the library 200 may then bind to target spots 110, such as illustrated in FIG. 2a. The non-binding members 206 of the library 200 may be partitioned or washed off the array 100. The binding and washing steps may be repeated and may also utilize an amplification step to generate additional copies of any remaining binding members 204 of the library 200. The array 100 may then be marked or tagged with a plurality of identifiers, such as, for example, a plurality of oligonucleotides which may universally bind through Watson-Crick interactions to the members of the library of, for example, nucleic acids. In one embodiment, each member 202 of the library 200 may include a potential binding sequence 202a and at least one conserved region 202b which may bind an identifier oligonucleotide, such as illustrated in FIG. 3. A further conserved region 202c may also be included to facilitate priming for amplification reactions, such as Polymerase Chain Reaction (PCR), as illustrated in FIG. 3a. In general the conserved regions 202b, 202c may flank the potential binding sequence 202a, such as to facilitate priming for amplification. An identifier 302 may then include a unique or semi-unique sequence 302a, such as illustrated in FIG. 3b, which may be utilized to correspond to the spots 110 and thus the targets of the array 100 by location of the a spot 110 on the substrate 102. The identifiers 302 may further include conserved region 302b which may bind to the conserved region 202b of the library members 202 by Watson-Crick base pairing. The identifiers 302 may also include a further conserved region 302c which may facilitate priming for amplification. The identifiers 302 may be, for example, applied to the spots 110 by printing, for example, inkjet printing, using micro-contact pins, and/or otherwise applying solutions containing identifiers 302 to the substrate 102 of the array 100, such as, for example, by pipetting or the like, onto the spots 110. A library member 202 bound to a target spot 110 may then be tagged with an identifier 302 via base pairing B at regions 202b, 302b, as illustrated in FIG. 4. Thus, the nucleic acids 202 bound to a particular target spot 110 may be identified by the sequence 302a of the identifier 302. In an exemplary embodiment, nucleic acid amplification, such as PCR, may be utilized to generate copies of the members 202 bound to the spots 110, incorporating the identifier sequence 302a (or more its complementary sequence) into the product 203, as illustrated in FIG. 4a. This may be desirable for associating a given member 202 with a target or targets 110 while preserving the particular sequence of the member 202. This may be particularly desirable for resolving multiple binders to a single target or members of the library that bind to multiple targets. Subsequent amplifications may utilize primers for the sequences 202c, 302c such that only the products 203 containing both the sequences 202a, 302a are amplified. It may be understood that references to nucleic acid sequences, such as above, may generally refer to either a particular sequence or the corresponding complementary nucleic acid sequence. In general, it may be desirable for single droplets and/or otherwise separated volumes of solutions containing identifiers 302 for each spot 110 on the array 100 such that the possibility of mistagging may be reduced.

In one aspect, the identifiers may be printed on all the targets. In another aspect, the identifiers may be printed only on targets with bound biomolecules.

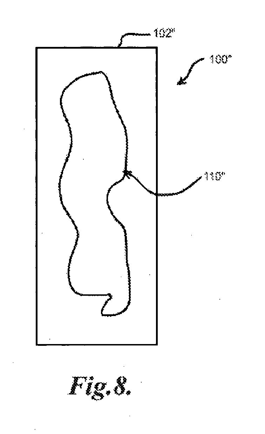

In another embodiment, a histology section, such as the section 110" on substrate 102" of histology slide 100" in FIG. 8, may be utilized as a target set. The section 110" may be, for example, a tissue section, a cell mass, and/or any other appropriate biological sample which may generally have structurally significant features. As with the array 100, a library of biomolecules, such as nucleic acids, may be applied which may bind to specific locations on the section 110", the locations on which may, for example, represent separate targets to generate affinity binding nucleic acids. Identifiers may then be disposed on the slide 100" as described above, or as in the embodiments below, such that identifiers may be utilized to correspond to specific features of the section 110".

In other embodiments, identifiers may be predisposed on the array substrate in substantial proximity to the spots, such as illustrated with identifiers 302 disposed on substrate 102 in proximity to spot 110 in FIG. 5, such that they may bind to nucleic acids bound to the target spots. The identifiers may, for example, be covalently attached to the substrate. In some embodiments, the attachments may be controllably breakable or cleavable such that the identifiers may be released from the substrate such that they may, for example, more easily bind to the bound nucleic acids on the spots.

In further embodiments, identifiers may be synthesized in situ on the array, such as by light directed in situ nucleic acid synthesis. Appropriately sequenced identifiers may then be synthesized in proximity to particular spots such that the newly synthesized identifiers may bind to the nucleic acids bound to the spot.

In still other embodiments, identifiers may be disposed and/or synthesized on a separate substrate, such as a membrane, in a spatial disposition that matches the spatial disposition of spots on the array. FIG. 5a illustrates an example of an identifier sheet 100' with membrane 102' which may include identifier spots 110' which may substantially correspond to target spots 110 of the array 100. The identifier sheet 100' may then be contacted C with the array 100 with locational matching of the target spots 110 with identifier spots 110'. The identifiers may then bind to the nucleic acids bound to the target spots. Any appropriate method of facilitating binding may be utilized, such as, for example, actions to drive migration of the identifiers to the array, such as capillary action, electrophoresis, pressure, gravitational settling, and/or any other appropriate method or combination thereof.

In some embodiments, the membrane may be soluble and/or substantially erodible. For example, the membrane may include a film forming and/or soluble material. Identifiers and/or other materials, such as components of a nucleic acid amplification or ligation reaction, may be included such that a film is formed containing the desired materials. The membrane may then be applied to the substrate and a suitable solvent, such as water or ethanol, may be utilized to dissolve and/or erode the film, which may then release the included materials, such as the identifiers, to the substrate. Suitable materials for the film may include hydrophilic materials including polysaccharides such as carrageenan, chondroitin sulfate, glucosamine, pullulan, soluble cellulose derivatives such as hydroxypropyl cellulose and hydroxymethyl cellulose, polyacrylic acid, polyvinyl alcohol, polyethylene glycol (PEG), polyethylene oxide (PEO), ethylene oxide-propylene oxide co-polymer, polyvinylpyrrolidone (PVP), polycaprolactone, polyorthoesters, polyphosphazene, polyvinyl acetate, and polyisobutylene.

The membrane may further be adapted to have a desirable rate of erosion and/or dissolution. The rate may be modified by the inclusion of hydrophobic and/or less soluble additives. Suitable materials may include, but are not limited to, those from the family of quaternary ammonium acrylate/methacrylate co-polymers, (Eudragit RS), cellulose and its lower solubility derivatives, such as butyl cellulose, hydroxybutyl cellulose and ethylhydroxyethyl cellulose, high molecular weight PEG or PEO or a combination thereof.

In yet still other embodiments, the array substrate may be physically divided and/or partitioned for separate collection of the nucleic acids bound to the spots. The spots may, for example, also be controllably removable from the substrate such that they may be individually recovered and sorted. The array itself may also be perforated and/or otherwise easily and/or conveniently partitionable.

In another embodiment, identifiers may be ligated to the bound nucleic acids. For example, a nucleic acid ligase may be utilized to covalently link an identifier sequence to the bound nucleic acid. In general, nucleic acid ligases are enzymes that covalently join two nucleic acids by catalyzing the formation of phosphodiester bonds at the ends of the phosphate backbone of the nucleic acids. Examples of appropriate nucleic acid ligases may include, but are not limited to, E. coli DNA ligase, T4 DNA ligase, T4 RNA ligase, strand break DNA repair enzymes, and/or any other appropriate ligase, modified enzyme, and/or a combination thereof. In general the ligase utilized may be selected based on the form of ligation performed, such as ligation of blunt ends, compatible overhang ("sticky") ends, single stranded DNA, singe stranded RNA and/or any other form of ligation. Further in general, the steps in ligating two nucleic acids together is a one step process that may be carried out at or near room temperature. Further nucleic acid fragments may be utilized to facilitate ligase action, such as appropriate complementary fragments that may aid the formation of a substantially double-stranded nucleic acid complex compatible with a ligase. In general, double stranded ligation may be employed and may utilize substantially compatible overhang fragments to facilitate ligation, or also blunt end ligation may be utilized, such as with either the nucleic acid end or the identifier having a phosphorylated end while the other is unphosphorylated for ligation. Single stranded ligation may also be employed.

Photo ligation may also be employed. Photo ligation may, for example, include covalently linking adjacent nucleic acids by application of electromagnetic energy, such as ultraviolet or visible light. Coupling agents may also be utilized to facilitate the formation of covalent linkages.

In some embodiments, dyes may be included into the identifiers. In one aspect, the identifiers may be doped with dyes. In another aspect, the identifier solutions may be mixed with dyes. According to one embodiment, the dyes may be photosensitive and may be fluorescent. According to another embodiment, the dyes maybe photosensitive and may be phosphorescent.

The substrates used may be glass, ceramic or polymeric, as long as their surfaces promote adhesion between the substrates and the targets. Polymers may include synthetic polymers as well as purified biological polymers. The substrate may also be any film, which may be non-porous or macroporous.

The substrate may be generally planar and may be of any appropriate geometry such as, for example, rectangular, square, circular, elliptical, triangular, other polygonal shape, irregular and/or any other appropriate geometry. The substrate may also be of other forms, such as cylindrical, spherical, irregular and/or any other appropriate form.

Appropriate ceramics may include, for example, hydroxyapatite, alumina, graphite and pyrolytic carbon.

Appropriate synthetic materials may include polymers such as polyamides (e.g., nylon), polyesters, polystyrenes, polyacrylates, vinyl polymers (e.g., polyethylene, polytetrafluoroethylene, polypropylene and polyvinyl chloride), polycarbonates, polyurethanes, poly dimethyl siloxanes, cellulose acetates, polymethyl methacrylates, ethylene vinyl acetates, polysulfones, nitrocelluloses and similar copolymers. These synthetic polymers may be woven or knitted into a mesh to form a matrix or similar structure. Alternatively, the synthetic polymer materials can be molded or cast into appropriate forms.

Biological polymers may be naturally occurring or produced in vitro by fermentation and the like or by recombinant genetic engineering. Recombinant DNA technology can be used to engineer virtually any polypeptide sequence and then amplify and express the protein in either bacterial or mammalian cells. Purified biological polymers can be appropriately formed into a substrate by techniques such as weaving, knitting, casting, molding, extrusion, cellular alignment and magnetic alignment. Suitable biological polymers include, without limitation, collagen, elastin, silk, keratin, gelatin, polyamino acids, polysaccharides (e.g., cellulose and starch) and copolymers thereof.

Any suitable substrate may be susceptible to adhesion, attachment or adsorption by targets. The susceptibility may be inherent or modified. In one example, the surfaces of substrates may be susceptible to adhesion, attachment or adsorption to proteins. In another example, the surfaces of substrates may be susceptible to adhesion, attachment or adsorption to proteins and not to nucleic acids.

In one exemplary embodiment, a glass substrate may have a layer or coating of a material that promotes adhesion with targets, such as proteins, materials that maybe charged, such as those that are positively charged, for binding target materials. Examples of charged materials include cellulosic materials, for example, nitrocellulose, methylcelluose, ethylcellulose, carboxymethyl cellulose, hydroxyethyl cellulose, methylhydroxypropyl cellulose; epoxies, PVDF (polyvinylidene fluoride); partially or fully hydrolyzed poly(vinyl alcohol); poly(vinylpyrrolidone); poly(ethyloxazoline); poly(ethylene oxide)-co-poly(propylene oxide) block copolymers; polyamines; polyacrylamide; hydroxypropylmethacrylate; polysucrose; hyaluronic acid; alginate; chitosan; dextran; gelatin and mixtures and copolymers thereof.

In another exemplary embodiment, if the substrate is not susceptible for attachment by charged materials, or may be susceptible only for attachment by wrongly charged materials, some areas of the substrate may have adhesives, binding agents, or similar attached, adsorbed or coated thereon. Examples of adhesives may include any suitable adhesives that bind the charged materials.

The targets may be present on the substrate discretely or in clusters. The distance between the discrete targets may be close or may be far apart and may usually be of different targets. Clusters may be used for multiple spots of a single target.

In one embodiment, the substrate may be macroporous. Macroporous substrates may be desirable, for example, if the different targets are very close together. When the targets are close by, there may not be sufficient distance between different targets to distinguish which target a biomolecule may be binding to. Closely packed targets may increase the efficiency of the generating of biomolecules. A macroporous substrate may be suited for balancing between efficiency and separation. For a macroporous substrate, the walls of the pores may be sufficient to separate even closely packed targets if the pores are large enough to enable the binding process to occur within the pores.

Also, for macroporous substrates, the pores may have an average diameter greater than the average size of the target material such that the target material may enter or partly enter the pores to anchor. Hydrogels may also be useful for binding or anchoring targets to the pores. Hydrogels may also fill the pores under fluid conditions and present a smooth surface for fluid flow while at the same time may keep the fluid from flowing through the pores.

The plurality of targets may be arranged in any appropriate manner such as, for example, in circular or elliptical spots, square or rectangular spots, stripes, concentric rings and/or any other appropriate arrangement on the subject.

According to one exemplary embodiment, the substrate may be at ambient temperature throughout.

According to another exemplary embodiment, the substrate may include a temperature affecting system that generally produces at least one desired temperature on the surface of the substrate and the adjacent fluid. The desired temperature may facilitate the biomolecule generating process.

According to a further exemplary embodiment, the substrate may include a temperature affecting system for producing a range of desired temperatures on the surface of the substrate and the adjacent fluid. This may be particularly useful when employing a set of targets having a significant range of, for example Tms, or melting temperatures. In one embodiment, the system may include a plurality of temperature affecting devices that are in thermal communication with the substrate. The plurality of devices may generally be disposed such that they may each produce a desired temperature in a given locality on the surface of the substrate. The set of targets may also be distributed on the surface of the substrate such that the temperature at the location of a target is substantially at the Tm of the target.

Temperature affecting devices may be any appropriate device that may substantially produce a desired temperature on a substrate and may include, but are not limited to, thermoelectric devices such as Peltier junction devices, semiconductor heating devices, resistive heating devices, inductive heating devices, heating/cooling pumps, electromagnetic radiation sources and/or any other appropriate devices. Temperature may also be affected by other systems, such as, for example, fluid flows including, but not limited to, water flows, air flows, and/or any other appropriate fluid flows.

In an exemplary embodiment, a plurality of Peltier junction devices may be utilized to generate desired temperatures at localities on the surface of the substrate. Peltier junction devices are particularly useful since they are able to both heat and cool using electrical current. This enables Peltier junction devices to generate temperatures above and below the ambient temperature of a system. They may also be useful in maintaining given temperature conditions at a steady state by adding and removing heat as necessary from the system.

In general, the placement of the temperature affecting devices may determine the temperature profile on the surface of the substrate and the adjacent fluid in the chamber. The temperature affecting devices may thus be disposed at appropriate positions such that given temperatures may be produced and maintained at known positions on the substrate.

The substrate may in general have a given thermal conductivity such that the application of at least one temperature affecting device may substantially generate a temperature gradient profile on the surface of the substrate. In general, the temperature on the surface of the substrate may change as a function of the distance from the position of the at least one temperature affecting device. Substrate materials with a relatively low thermal conductivity may generally produce highly localized temperature variations around a temperature affecting device. Substrate materials with a relatively high thermal conductivity may generally produce more gradual variations in temperature over a given distance from a temperature affecting device. It may be understood that at steady state, the effect of the thermal conductivity of the substrate may not contribute to the temperature profile of the system.

In some embodiments, at least one temperature affecting device may be utilized to produce a particular temperature gradient profile on the surface of the substrate. In general, a temperature gradient may be generated by utilizing at least one temperature affecting device producing a temperature different from the ambient temperature of the system. Multiple temperature affecting devices with at least two producing different temperatures may be utilized to generate a temperature gradient without reliance on the ambient temperature of the system.

The positions and temperatures of multiple temperature affecting devices may be utilized to calculate a resulting temperature gradient profile on the surface of a substrate using standard heat transfer equations. An algorithm may then be utilized to calculate the optimal positions and/or temperatures for a plurality of temperature affecting devices to produce a desired temperature gradient profile on the surface of a substrate. The algorithm may be, for example, applied using a computational assisting system, such as a computer and or other calculatory device. This may be performed to tailor a temperature gradient profile to a particular substrate with a known disposition of targets of known and/or calculated Tm. Similarly, a set of targets of known and/or calculated Tm may be arranged on a substrate based on a temperature gradient profile. This may be desirable as placement of a target at a given location on a substrate may be accomplished more easily than tailoring a temperature profile to pre-existing locations of targets on a substrate. In general, a target may be disposed on the substrate at a temperature address within the temperature profile gradient. The temperature address may, for example, be substantially at the Tm of the target during operation of the molecular hybridization system, and/or any other appropriate temperature.

In another aspect, the molecular hybridization system includes an adjustable system for generating a temperature profile. The adjustable system generally includes a plurality of temperature affecting devices, each affecting the temperature at a particular location of a substrate.

Details of the temperature affecting systems may be found in, for example, U.S. utility patent application Ser. No. 12/249,525, filed on October 10, 2008, entitled "METHODS AND DEVICES FOR MOLECULAR ASSOCIATION AND IMAGING".

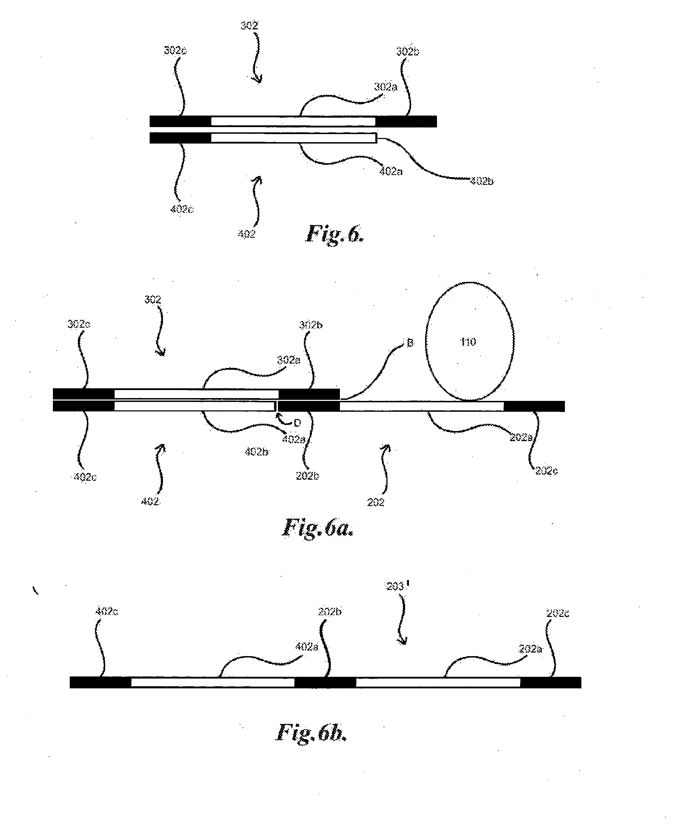

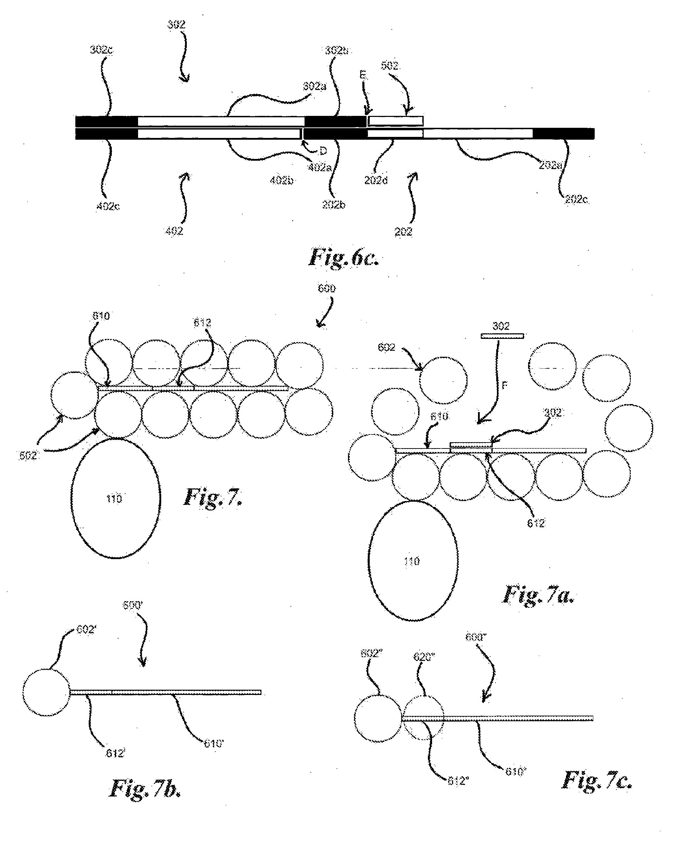

FIG. 6 illustrates an example of an identifier sequence 302 and a complement identifier sequence 402. The complement sequence 402 may include a complement identifier region 402a which may be substantially complementary to identifier region 302a such that they may base pair bind. The complement sequence 402 may further include a primer region 402c which may also be complementary to primer region 302c of the identifier 302. Further, the complement sequence 402 may include a compatible end 402b which may be compatible with ligation to the end of another nucleic acid. As shown in FIG. 6a, a nucleic acid library member 202 may be bound to a spot 110. An identifier 302 and a complement sequence 402 may then be applied to the member 202 such that the identifier 302 binds to the member 202 at region 202b, 302b. The complement sequence 402 may bind to the identifier 302 at regions 302a/402a, 302c/402c. The compatible end 402b may then be ligated to the end D of the member 202 by an appropriate ligase and/or other appropriate method. A product 203', as illustrated in FIG. 6b, may then be generated including the primer region 202c, binding sequence 202a, region 202b, complement identifier region 402a, and complement primer region 402c. The product 203' may then be amplified, such as with the product 203 discussed above in FIG. 4a. The product 203' may also be generated by single-stranded ligation of the member 202 and the complement sequence 402, where in general the either the member 202 or the complement sequence 402 may have a phosphorylated end while the other may be unphosphorylated for end to end ligation.

In another example, as illustrated in FIG. 6c, a further complementary fragment 502 may be included that may base pair bind to a complementary region 202d of the nucleic acid library member 202. This may be desirable as some nucleic acid ligases may generally join double stranded nucleic acids. The addition of the complementary fragment 502 may generally generate a substantially double stranded nucleic acid, such as illustrated spanning from region 302c to the end of complementary fragment 502. There may further be a double stranded "break" at points D and E. In general, the sizing of the fragments may be tailored to generate a suitably long stretch of double stranded nucleic acid for ligase action. In general, the complementary region 202d may be the same for all members 202 of the library 200 such that the same complementary fragment 502 may be utilized, such as, for example, convenience, cost and/or ease of use.

In general, methods may be applied that may facilitate binding or other interactions between the identifiers and the nucleic acids bound to the spots. For example, the temperature may be increased to dissociate the nucleic acids from the spots. The temperature may subsequently be lowered such that, for example, base pairing may occur between the nucleic acids and the identifiers. Temperature changes may also, for example, denature the target such that the nucleic acids may no longer bind and/or bind with lower affinity to the targets. This may be desirable in that it may aid in binding of the nucleic acids to the identifiers.

In a further aspect of the invention, methods for monitoring and/or controlling the diversity of the library of biomolecules may be utilized. For example, too few rounds of selection may result in a biomolecule pool with too many weak binding members while too many rounds of selection may result in only a few binding members, such as members corresponding to only a few targets rather than members corresponding to all of the targets present. In one embodiment, Cot analysis may be employed to measure and/or monitor the diversity of the library of biomolecules through multiple rounds of selection. Cot, or Concentration x time, analysis measures the annealing time of particular oligonucleotides while in solution with other nucleic acids, such as the members of the library of biomolecules. In general, the annealing time will be faster the lower the diversity of the library.

In one embodiment, a Cot-standard curve for measuring the sequence diversity of the aptamer library at any point during the multiplex SELEX process may be utilized. For example, a group of DNA oligonucleotides with a 5'- and 3'-constant region of ∼20 bases identical to the initial SELEX library may be utilized. The oligos may then be converted to dsDNA by standard methods. Briefly, after annealing a primer to the oligos, Exo-minus Klenow Taq polymerase (Epicentre, Madison, WI) may be used in conjunction with dNTPs to fill in the ssDNA to create a dsDNA or mixture thereof. Using a standard quantitative PCR thermal cycler, a temperature profile for melting and controlled annealing of each DNA mixture may be programmed. Standard SYBR Green I specific for double-stranded DNA (dsDNA) may be utilized to report the amount of re-annealed dsDNA. At one extreme, the annealing time for a single sequence will be measured. At the other extreme, the annealing time for the initial SELEX pool, such as containing approximately 1 nmol of sequence diversity, may be measured. Annealing times of intermediate diversity may also be measured to establish a very specific Cot-standard-curve for the SELEX library. Using this standard curve, at any point during SELEX, the sequence diversity of the evolving library of aptamers may be determined by comparison to the curve.

In a further aspect, a method for generating functional biomolecules includes obtaining a library of peptide sequences and contacting the library with a plurality of targets. In some aspects, the peptide sequence may be tagged, linked, marked and/or otherwise associated with a nucleic acid sequence. The nucleic acid sequence may be, for example, representative of the sequence of the peptide. For example, the nucleic acid may substantially encode the peptide sequence. Also for example, the nucleic acid may be a unique or semi-unique identifier sequence. The nucleic acid sequence may then be utilized to bind another identifier, as described above, such that a peptide bound to a target may be tagged or marked as to which target it bound.

In an exemplary aspect, a bacteriophage (phage) may be generated that includes a peptide sequence of interest in its protein coat. The phage may further include a nucleic acid sequence that may be representative of the peptide sequence within the nucleic acid of the phage. The phage may then be contacted with a plurality of targets, as above. This may generally be referred to as phage display. Phages employed may include, but are not limited to, M13 phage, fd filamentous phage, T4 phage, T7 phage, λ phage, and/or any other appropriate phage. Non-binding phages may be washed and/or partitioned, while binding phages may be tagged or marked with identifiers, as above. As phage nucleic acids are generally contained within the protein coat of the phage, the nucleic acid may generally be exposed for binding to the identifier. For example, the phage may be heated such that the protein coat denatures and/or disassembles such that the nucleic acid is exposed. The identifier may also be introduced into the phage, such as by electroporation, electrophoresis, and/or any other appropriate method.

In FIG. 7, an example of a phage 600 may include a nucleic acid 610 which may generally encode, among other things, and be encapsulated by a protein coat 602, which may contain a binding region for a target 110. The nucleic acid 610 may further include a region 612 which may identify the phage and/or encode the binding region for a target. A bound phage 600, as illustrated in FIGs. 7 and 7a, may then be heated, disrupted and/or otherwise treated such that an identifier 302 may contact F the region 612. For example, the protein coat 602 may be broken and/or otherwise disrupted for entry of the identifier 302. In general, an amplification reaction and/or other method, such as those discussed above, may be utilized to tag, mark and/or otherwise introduce identifier information to the sequence of region 612. Further in general, the identifier 302 and region 612 may incorporate any, all or a combination of the elements discussed above in regards to nucleic acid library members, identifiers and/or other nucleic acid fragments. As also discussed above, the phage 600 may also be physically removed and/or partitioned in a manner that may preserve the identity of the target 110 the phage 600 was associated.

In other aspects, other methods of incorporating and/or linking nucleic acids to peptides may be utilized, such as, for example, mRNA display, ribosome display, and/or any other appropriate method. In general, in mRNA display, as illustrated in FIG. 7b, a fusion product 600' of a messenger RNA (mRNA) 610' may be linked to a peptide 602' that the mRNA 610' encodes, such as with a puromycin-ended mRNA 612' which may generally cause fusion of the mRNA 610' to the nascent peptide 602' in a ribosome, which may then be contacted with targets such as described above with phage display. Also in general, in ribosome display, as illustrated in FIG. 7c, a fusion product 600" of a modified mRNA 610" may be utilized that codes for a peptide 602", but lacks a stop codon and may also incorporate a spacer sequence 612" which may occupy the channel of the ribosome 620" during translation and allow the peptide 602" assembled at the ribosome 620" to fold, which may result in the peptide 602" attached to the ribosome 620" and also attached to the mRNA 610". This product 600" may then be contacted with targets such as described above with phage display. Other methods may include, but are not limited to, yeast display, bacterial display, and/or any other appropriate method.

In another aspect of the disclosure, methods for handling and sorting the resultant sequences of a multiplexed binding process are provided. In some embodiments of the disclosure, the sequences may be sorted by identifier sequences to establish which target or targets the sequence bound. The sequences may further be compared, aligned and/or otherwise processed to identify features, characteristics and/or other useful properties, relationships to each other, and/or target properties. For example, it may be expected that multiple aptamer sequences bound to a single target may potentially share sequence motifs and/or other common features which may be at least partially elucidated by sequence sorting and/or comparison. Specific binding affinities of resultant sequences may also be determined through affinity assays. In some embodiments, surface plasmon resonance may be utilized to determine binding of an aptamer to a target. For example, sensors which monitor the refractive index of a surface bound to a target may be utilized, where the refractive index may change as a result of binding of an aptamer to the target. In general, aside from standard sequencing methods, parallel sequencing methods, such as, for example, massively parallel sequencing such as 454 Clonal Sequencing (Roche, Branford, CT), massively parallel clonal array sequencing, Solexa Sequencing (Illumina, San Diego, CA), and/or any other appropriate sequencing method may be employed.

### EXAMPLE OF MULTIPLEXED SELEX PROTOCOL

As a demonstration of parallel, de novo selection of aptamers against multiple targets, a combinatorial DNA library containing a core randomized sequence of 40 nts flanked by two 20 nt conserved primer binding sites is used as the starting point for an aptamer pool. The primer sequences are designed and optimized using Vector NTI's (Invitrogen) oligo analysis module. Typically, such a library is expected to contain approximately 10¹⁵ unique sequences. The primer binding sites are used to amplify the core sequences during the SELEX process. The single stranded DNA pool dissolved in binding buffer is denatured by heating at 95°C for 5 min, cooled on ice for 10 min and exposed to multiple protein targets fixed onto a nitrocellulose coated glass slide (e.g., Whatman).

### EXAMPLE OF DNA LIBRARY SELEX

An example DNA library consists of a random sequence of 40 nucleotides flanked by conserved primers with the following sequence: (Forward): 5'-ATACCAGCTTATTCAATT-3', and reverse primer (RP): 5'-AGATTGCACTTACTATCT-3'. In the first round of SELEX, 500 pmol of the ssDNA pool is incubated with each slide in binding buffer (PBS with 0.1 mg/ml yeast tRNA and 1 mg/ml BSA) for 30 minutes at 37°C. The slide is then washed in 1 ml of binding buffer for one minute. To elute specifically bound aptamers the slide is heated to 95°C in binding buffer. The eluted ssDNA is subsequently be precipitated using a high salt solution and ethanol. After precipitation, the aptamer pellet is resuspended in water and amplified by PCR with a 3'- biotin-labeled primer and a 5'-fluorescein (FITC)-labeled primer (20 cycles of 30 sec at 95°C, 30 sec at 52°C, and 30 sec at 72°C, followed by a 10 min extension at 72°C). The selected FITC-labeled sense ssDNA is separated from the biotinylated antisense ssDNA by streptavidin-coated Sepharose beads (Promega, Madison, WI) for use in the next round. Alternatively, "asymmetric PCR" may be utilized for generating a large excess of an intended strand of a PCR product in SELEX procedures. Also alternatively, the undesired strand may be digested by λ-exonuclease, such as, for example, when a phosphorylated PCR primer is employed.

The labeling of individual aptamers with fluorescein isothiocyanate (FITC) facilitates the monitoring of the SELEX procedure. FITC is also compatible with scanning in the green (cy3) channel of standard microarray scanners. The sense primer used to amplify the ssDNA aptamers after each round of selection is fluorescently labeled, resulting in fluorescently labeled aptamers. The protein spotted nitrocellulose-coated slides are scanned in a microarray scanner. Alternatively, proteins may be spotted on epoxy-coated glass slides. While epoxy slides may have less protein binding capacity than 3-D nitrocellulose pads, it has been observed that there may be less non-specific binding of nucleic acid aptamer pools to the background of the slide (blocked or not). Blocking may be employed to reduce background fluorescence.

In each round of the SELEX process, the slide is incubated for 30 min at 37°C to allow binding of the aptamers to their targets. The slides are then washed in binding buffer before the specifically bound DNAs are eluted by heating the slide at 95°C in 7M urea. Nucleic acids from the eluate are phenol-chloroform purified and precipitated, and the concentrated single stranded DNA molecules will be amplified by PCR. In order to increase stringency throughout the SELEX process, the washes are gradually increased in volume (from approximately 1-10 ml). After a given point in the selection, such as, for example, after the final round of selection, the aptamers may be tagged, marked and/or partitioned.

### EXAMPLE OF IN SITU HYBRIDIZATION OF IDENTIFIERS

An example of in situ hybridization of identifiers to aptamers was performed with short, ssDNA sequence tags to the 3' end of aptamers bound to their protein target. These synthetic ssDNA tag oligonucleotides consists of three regions, as illustrated in FIG. 3b with identifier 302: (i) the C2 region, region 302c of the identifier 302, at the 3' end of the oligonucleotide consists of a 17-20 nucleotide sequence complementary to a corresponding region on all of the used aptamers, (ii) the C1 region, region 302b at the 5' end of the oligonucleotide 302 contains a 17-20 nt primer binding site, used during the amplification of the tag:aptamer hybrid, prior to sequencing and (iii) a variable region 302a in the center of the tag oligonucleotide (V) that serves a as a unique identifier for each locus on the glass slide surface. A variable sequence of 8 nucleotides will allow 48 (65,536) unique sequences to be generated, sufficient for many complex protein arrays (8000 samples) on the market.

As outlined above, after the final round of the SELEX procedure (typically, round 10) the specific aptamers are bound to their protein targets, fixed to a glass slide. While the 40 nt core sequence of each aptamer are unique, its terminal sequences have not been subject to any kind of selection during the procedure. After each round of binding to their protein targets, the aptamers were amplified using conserved primers, requiring the maintenance of corresponding regions at their distal ends (P1, P2). The 3'-region of each aptamer, for instance, can thus serve as a binding site (via standard hybridization) for the C2 region of the proposed tag oligonucleotide. Given the unique variable sequence (V) of each tag oligonucleotide, each aptamer will now be tagged with a sequence, that can be traced back to the location of the aptamer on the glass slide, and thus the protein spotted at that location.

### EXAMPLE OF 16-PLEX SELEX PROCEDURE

An example of a 16-plex (16 targets) SELEX procedure was performed with 16 unique targets: (1) fibrinogen, (2) collagen, (3) fibronectin, (4) acetyl-bovine serum albumin (BSA), (5) heparan sulfate, (6) prolactin inhibiting factor (PIF), (7) ribonuclease A (RNase A), (8) laminin, (9) interleukin-7 cat 200-7 (IL-7 cat 200-7), (10) IL-15 cat 200-15, (11) IL-21 cat 200-21, (12) IL-7R cat 306-IR, (13) IL-15R, (14) IL-21R, (15) IgG2a (anti-CD19), and (16) anti-CD20. The 16 targets were arranged as a spotted array on a blocked nitrocellulose slide. A DNA aptamer library as discussed above was applied to the slide. Non-binding members of the library were washed off and the slide was labeled with 16 unique identifier nucleic acid sequences, one per target spot, as described above. The identifiers were briefly incubated at 60° C and then allowed to hybridize to the aptamers on the spots at 37° C for 30 minutes. Exo-Klenow Taq polymerase and dNTPs were added and extension of the identifiers was performed for 1 hour at 37° C. The Taq was inactivated at 60° C for 10 minutes. The dsDNA was eluted with near boiling 7M urea and then precipitated with 3M sodium acetate at pH 5.0 and ice cold ethanol. The recovered dsDNA was sequenced by standard methods.

Identifier sequences in the sequenced DNA were utilized to identify the target of the aptamer sequence. The following aptamer sequences were identified for the targets (identifier sequences and priming sequences removed):

Target 1:
5'-CAAGAGTGTTAGACATTATCTCAGCGCTGCCAATTATATT-3' (Seq1)

Target 2:
5'-AGAGGCGGCTGAGATCAATCTCCGCTCAGGGAGCGAGTA-3' (Seq2)

Target 3:
5'-CAATACAATACTATATTTGTGTCAATCTCGTACTTCTGAC-3' (Seq3)
5'-CAATATGTCTAATTTTTTTACATGGCGGCATGGTATTGGC-3' (Seq4)

Target 4:
5'-AAGATCTTTATTAAGCAAACAATGTTAACTATAGAGCGTT-3' (Seq5) 5'-GAATTACATTCAAAAATTTTCTTCTGGCATCTGTAATACCG-3' (Seq6)

Target 5:
5'-TGATACAAATACTCTCAATCAAAGCCAATATGTCGCAAAA-3' (Seq7)
5'-CAAAGTAAAATTAACAGATAGTACGTTCTCAATCTCGCGA-3' (Seq8)

Target 6:
5'-TCTTCTTGCACATATTTTTCTCCGTGAGACATGTAAATAA-3' (Seq9)
5'-ATGTACTTCACTTCAGTTTTCTTTAAACACGTTTCACATA-3' (Seq10)
5'-CCGCATTTATCAGTTTACCGCCCCATAAACATAACCGCT-3' (Seq11)
5'-AATTATGCTCATGATTTTCTTCAAAAAGGCTCGCGCAATT-3' (Seq12)
5'-GGCTGTTAAACTTACTTTTCTTCAGTAATTGCCGTTGACA-3' (Seq13)
5'-TAGTTTATCAGGAGCGATCACTGATCATGAGTAACTTTTA-3' (Seq14)
5'-ATCAAGAATTGATAATTTTAGGAATTGCGTATCGCTGCTA-3' (Seq15)

Target 7:
5'-AACTGTGTTTTAGGACTTCATTGTCTTAATTCTCTTCCCT-3' (Seq16)
5'-AGCGCGATATTACGGTCTCGAACCAAAACCATCACGGTTC-3' (Seq17)
5'-ATAGTTTAAATTTAATCTTCTGCCACCCTTCACTTTCA-3' (Seq18)

Target 8:
5'-GCACATCTCCCGTTCGACTTTTTTATCTTCGAGCACCTAA-3' (Seq19)
5'-CCATGTAGCACAAAACAACGATATAAGAACTACATTTAGT-3' (Seq20)
5'-GGCTTTCTAATCTAACACGATCTCCTCTCCTTACGCCGTG-3' (Seq21)
5'-TACTCTTGTTTAAACTAGAACAGTAAAATATTAATTCTTA-3' (Seq22)
5'-TGGAGTTTATAATACTCGAGGCTAGTAGTGCCATTTTACA-3' (Seq23)
5'-ACGCGCGGCTGTGGGGAAGGTACAGGTTCCGAACGATGGA-3' (Seq24)
5'-ACAAGAACTATTTTTATCAAAGACGTCACCAACTTAAGGC-3' (Seq25)
5'-CAAATAATCGTTTTATAATTACCAACACATTTTGGTTAAC-3' (Seq26)
5'-TTTATATTAAGCAACTTTTTGAGAGTTGATTGATAATTTA-3' (Seq27)

Target 9:
5'-TGGCCTAATCTCGGAGACTGGCCGCTGTGGGCGCGGGCCT-3' (Seq28)

Target 10:
5'-TCTAATTATGTTACAAAATAATTGTTATGCTCCGCAAATA-3' (Seq29)
5'-TCATTTAATTGTCCTAAATCTGAAAATTTATTATATTTC-3' (Seq30)

Target 11:
5'-CCGGGATAAATTACTAAGTTTCGGGTATTTTGACAATATT-3' (Seq31)
5'-AAACCTGCAAAATTTAGGGCCAATGTGTGTATTGAACGGG-3' (Seq32)
5'-GTTATAGTAATATTGGTTCTAGCTCTCAGTAATATCAAAA-3' (Seq33)
5'-ACCCTATAAGCTGAGATAAGCATTCTGTGGACGAAAAGTT-3' (Seq34)

Target 13:
5'-AGGCGTTAACTCTTGTCATGTTATAGACGTCTAATCCATC-3' (Seq35)

Target 14:
5'-ACTGTAATCACTTCTTTTAAATAGTCCCGGAACGATATCA-3' (Seq36) 5'-CTTGATCCATACTATAACTTAACATTTGTTCATCTCAAGT-3' (Seq37)

Target 15:
5'-CTAAATCGCGAACCGAGTTTTTGTCAAAGTTCTAGATTAA-3' (Seq38)

Target 16:
5'-ACGAACTTTTTTAATTCGCAGACATGTTTATAGTTTCTTG-3' (Seq39)

No aptamers were recovered for target 12 (IL-7R cat 306-IR).

## Claims

1. A method for simultaneously selecting a plurality of different aptamers for multiple targets comprising the following steps:
a) contacting a library of aptamers with a plurality of targets simultaneously, wherein the plurality of targets are affixed to a substrate within a single volume and disposed in the form of an array of spots with each target identifiable by the location of a particular spot on the substrate, wherein each member of the library includes: a potential target-binding sequence, at least one conserved region capable of binding to an identifier oligonucleotide by hybridization, and a further conserved region for priming nucleic acid amplification reactions;
b) partitioning members of said library that do not bind to said plurality of targets;
c) marking the members of the library that bind target spots with identifier oligonucleotides, where each identifier oligonucleotide includes: a unique sequence that is utilized to correspond to the location of a target on the array, a conserved region capable of binding to the conserved region of the library members by hybridisation, and a further conserved region for priming nucleic acid amplification reactions; and
d) identifying the binding members of the library and correlating them to the targets via the unique sequence of the identifier utilizing nucleic acid amplification to generate copies of the binding members with the unique sequence of the identifier oligonucleotide incorporated into the amplified product.

2. The method of claim 1, wherein the single reaction volume includes multiple reaction sub-volumes.

3. The method of claim 1 or 2 wherein the plurality of targets are disposed on a substrate with multiple copies of at least one target.

4. The method of claim 1, 2 or 3, wherein the partitioning comprises washing.

5. The method of claim 4 further comprising repeating the contacting and washing steps.

6. The method of any of claims 1-5, wherein the contacting step comprises manual pipetting, automatic pipetting, inkjet printing, applying micro-contact pins or applying a membrane with identifiers disposed thereon.

7. The method of any of claim 1-6, further comprising attaching, ligating, photoligating, or covalently bonding the identifiers to the aptamers.

8. The method of any of claims 1-7 further comprising performing at least one round of a SELEX protocol with the library of aptamers against multiple targets prior to or after contacting with the plurality of targets on the substrate.

9. A system for performing the method of claim 1 comprising:
a) the library as specified in claim 1;
b) the plurality of targets as specified in claim 1; and
c) the plurality of identifiers as specified in claim 1.

10. The system of claim 9, wherein the array of spots disposed on the substrate has a spatial arrangement

11. The system of claim 9 or 10, wherein the identifiers comprise nucleic acids having unique or semi-unique sequences that correspond to the plurality of targets.

12. The system of claim 9, 10 or 11, wherein the identifiers comprise primers for the bound aptamers.

13. The system of claim 9, 10, 11 or 12, wherein the identifiers are synthesized in situ on the substrate.

14. The system of claim 9, 10, 11, 12 or 13, wherein the identifiers are disposed on a separate substrate matching the same spatial arrangement as the disposition of targets on the substrate.

## Patentansprüche

1. Verfahren zum simultanen Auswählen einer Vielzahl von unterschiedlichen Aptameren für mehrere Zielstrukturen, wobei das Verfahren die folgenden Schritte aufweist:
a) simultanes In-Kontakt-Bringen einer Bibliothek an Aptameren mit einer Vielzahl an Zielstrukturen, wobei die Vielzahl an Zielstrukturen in einem einzigen Volumen an ein Substrat fixiert und in Form eines Arrays an Spots aufgebracht ist, wobei jede Zielstruktur durch die Position eines bestimmten Spots auf dem Substrat identifizierbar ist, und wobei jedes Element der Bibliothek Folgendes einschließt: eine potenzielle Zielstruktur-bindende Sequenz, zumindest eine konservierte Region, die dazu in der Lage ist, durch Hybridisierung an ein Identifizierer-Oligonucleotid zu binden, und eine weitere konservierte Region für den Start von Nucleinsäureamplifikationsreaktionen;
b) Abtrennen von Elementen der Bibliothek, die nicht an die Vielzahl von Zielstrukturen binden;
c) Markieren der an die Zielstruktur-Spots bindenden Elemente der Bibliothek mit Identifizierer-Oligonucleotiden, wobei jedes Identifizierer-Oligonucleotid Folgendes einschließt: eine spezifische Sequenz, die dazu eingesetzt wird, der Position einer Zielstruktur auf dem Array zu entsprechen, eine konservierte Region, die dazu in der Lage ist, durch Hybridisierung an die konservierte Region der Elemente der Bibliothek zu binden, und eine weitere konservierte Region für den Start von Nucleinsäureamplifikationsreaktionen; und
d) Identifizieren der bindenden Elemente der Bibliothek und deren Korrelieren bezüglich der Zielstrukturen mittels der spezifischen Sequenzen der Identifizierer, und zwar unter Einsatz von Nucleinsäureamplifikation, um Kopien der bindenden Elemente zu generieren, wobei die spezifischen Sequenzen der Identifizierer-Oligonucleotide im amplifizierten Produkt eingebaut sind.

2. Verfahren nach Anspruch 1, bei welchem das einzige Reaktionsvolumen verschiedene Reaktions-Sub-Volumen mit einschließt.

3. Verfahren nach Anspruch 1 oder 2, in welchem die Vielzahl an Zielstrukturen mit verschiedenen Kopien mindestens eines Targets auf einem Substrat verteilt sind.

4. Verfahren nach Anspruch 1, 2 oder 3, bei welchem das Abtrennen Waschen umfasst.

5. Verfahren nach Anspruch 4, das ferner ein Wiederholen der Schritte des In-Kontakt-Bringens und Waschens umfasst.

6. Verfahren nach einem der Ansprüche 1-5, bei welchem das In-Kontakt-Bringen Folgendes umfasst: manuelles Pipettieren, automatisches Pipettieren, Inkjet-Drucken, Anwenden von Mikro-Kontaktpins oder Anwenden einer Membran mit hierauf aufgebrachten Identifizierern.

7. Verfahren nach einem der Ansprüche 1-6, das ferner das Anbringen, Ligieren, Photoligieren oder das kovalente Binden der Identifizierer an die Aptamere umfasst.

8. Verfahren nach einem der Ansprüche 1-7, das ferner die Durchführung von zumindest einer Runde eines SELEX-Protokolls mit der Bibliothek an Aptameren gegen verschiedene Zielstrukturen umfasst, und zwar vor oder nach In-Kontakt-Bringen mit der Vielzahl an Zielstrukturen auf dem Substrat.

9. System zur Durchführung des Verfahrens nach Anspruch 1, das Folgendes aufweist:
a) die in Anspruch 1 definierte Bibliothek;
b) die Vielzahl an Zielstrukturen wie in Anspruch 1 definiert; und
c) die Vielzahl an Identifizierern wie in Anspruch 1 definiert.

10. System nach Anspruch 9, in welchem das Array an auf dem Substrat aufgebrachten Spots eine räumliche Anordnung aufweist.

11. System nach Anspruch 9 oder 10, in welchem die Identifizierer Nucleinsäuren mit spezifischen oder halb-spezifischen Sequenzen umfassen, welche der Vielzahl an Zielstrukturen entsprechen.

12. System nach Anspruch 9, 10 oder 11, in welchem die Identifizierer Primer für die gebundenen Aptamere umfassen.

13. System nach Anspruch 9, 10, 11 oder 12, in welchem die Identifizierer *in situ* auf dem Substrat synthetisiert werden.

14. System nach Anspruch 9, 10, 11, 12 oder 13, in welchem die Identifizierer auf einem separaten Substrat aufgebracht sind, das bezüglich der gleichen räumlichen Anordnung wie die Aufbringung der Zielstrukturen auf dem Substrat abgestimmt ist.

## Revendications

1. Procédé pour sélectionner simultanément une pluralité d'aptamères différents pour de multiples cibles, comprenant les étapes suivantes :
a) la mise en contact d'une banque d'aptamères avec une pluralité de cibles simultanément, dans laquelle la pluralité de cibles sont fixées à un substrat dans un volume unique et disposées sous la forme d'un alignement de points, chaque cible étant identifiable par l'emplacement d'un point particulier sur le substrat, dans laquelle chaque élément de la banque inclut : une séquence se liant à une cible potentielle, au moins une région conservée capable de se lier à un oligonucléotide identifiant par hybridation, et une autre région conservée pour amorcer les réactions d'amplification des acides nucléiques ;
b) la séparation des éléments de ladite banque qui ne se lient pas à ladite pluralité de cibles ;
c) le marquage des éléments de la banque qui se lient à des points de cibles avec des oligonucléotides identifiants, où chaque oligonucléotide identifiant inclut : une séquence unique qui est utilisée pour correspondre à l'emplacement d'une cible sur l'alignement, une région conservée capable de se lier à la région conservée des éléments de la banque par hybridation, et une autre région conservée pour amorcer les réactions d'amplification des acides nucléiques ; et
d) l'identification des éléments de liaison de la banque et la corrélation de ceux-ci aux cibles par l'intermédiaire de la séquence unique de l'identifiant en utilisant une amplification des acides nucléiques pour générer des copies des éléments de liaison ayant la séquence unique de l'oligonucléotide identifiant incorporée dans le produit amplifié.

2. Procédé selon la revendication 1, dans lequel le volume de réaction unique inclut de multiples sous-volumes de réaction.

3. Procédé selon la revendication 1 ou 2, dans lequel la pluralité de cibles sont disposées sur un substrat avec de multiples copies d'au moins une cible.

4. Procédé selon la revendication 1, 2 ou 3, dans lequel la séparation comprend un lavage.

5. Procédé selon la revendication 4, comprenant en outre la répétition des étapes de mise en contact et de lavage.

6. Procédé selon l'une quelconque des revendications 1 à 5, dans lequel l'étape de mise en contact comprend le pipetage manuel, le pipetage automatique, l'impression par jet d'encre, l'application de broches micro-contact ou l'application d'une membrane avec des identifiants disposés sur celle-ci.

7. Procédé selon l'une quelconque des revendications 1 à 6, comprenant en outre l'attachement, la ligature, la photoligature, ou la liaison covalente des identifiants aux aptamères.

8. Procédé selon l'une quelconque des revendications 1 à 7, comprenant en outre la réalisation d'au moins un tour d'un protocole SELEX avec la banque d'aptamères contre de multiples cibles avant ou après la mise en contact avec la pluralité de cibles sur le substrat.

9. Système pour mettre en oeuvre le procédé selon la revendication 1, comprenant :
a) la banque telle que spécifiée dans la revendication 1 ;
b) la pluralité de cibles telles que spécifiées dans la revendication 1 ; et
c) la pluralité d'identifiants tels que spécifiés dans la revendication 1.

10. Système selon la revendication 9, dans lequel l'alignement de points disposés sur le substrat a un agencement spatial.

11. Système selon la revendication 9 ou 10, dans lequel les identifiants comprennent des acides nucléiques ayant des séquences uniques ou semi-uniques qui correspondent à la pluralité de cibles.

12. Système selon la revendication 9, 10 ou 11, dans lequel les identifiants comprennent des amorces pour les aptamères liés.

13. Système selon la revendication 9, 10, 11 ou 12, dans lequel les identifiants sont synthétisés *in situ* sur le substrat.

14. Système selon la revendication 9, 10, 11, 12 ou 13, dans lequel les identifiants sont disposés sur un substrat séparé ayant le même agencement spatial que la disposition des cibles sur le substrat.


### SEQUENCE LISTING

<110> Jackson, George
<120> METHODS FOR SIMULTANEOUS GENERATION OF FUNCTIONAL LIGANDS
<130> P1011US01
<140> 12/XXX,XXX
   <141> 2010-01-06
<150> 61/162,394
   <151> 2009-03-23
<150> 12/479,806
   <151> 2009-06-06
<160> 41
<170> PatentIn version 3.5
<210> 1
   <211> 18
   <212> DNA
   <213> Artificial Sequence
<220>
   <223> DNA Amplification Priming Sequence (artificial sequence)
<400> 1
   ataccagctt attcaatt 18
<210> 2
   <211> 18
   <212> DNA
   <213> Artificial Sequence
<220>
   <223> DNA Amplification Priming Sequence (artificial sequence)
<400> 2
   agattgcact tactatct 18
<210> 3
   <211> 40
   <212> DNA
   <213> Artificial Sequence
<220>
   <223> DNA aptamer sequence for fibrinogen (artificial)
<400> 3
   caagagtgtt agacattatc tcagcgctgc caattatatt 40
<210> 4
   <211> 39
   <212> DNA
   <213> Artificial Sequence
<220>
   <223> DNA aptamer sequence for collagen (artificial)
<400> 4
   agaggcggct gagatcaatc tccgctcagg gagcgagta 39
<210> 5
   <211> 40
   <212> DNA
   <213> Artificial Sequence
<220>
   <223> DNA aptamer sequence for fibronectin (artificial)
<400> 5
   caatacaata ctatatttgt gtcaatctcg tacttctgac 40
<210> 6
   <211> 40
   <212> DNA
   <213> Artificial Sequence
<220>
   <223> DNA aptamer sequence for fibronectin (artificial)
<400> 6
   caatatgtct aattttttta catggcggca tggtattggc 40
<210> 7
   <211> 40
   <212> DNA
   <213> Artificial Sequence
<220>
   <223> DNA aptamer sequence for acetyl BSA (artificial)
<400> 7
   aagatcttta ttaagcaaac aatgttaact atagagcgtt 40
<210> 8
   <211> 41
   <212> DNA
   <213> Artificial Sequence
<220>
   <223> DNA aptamer sequence for acetyl BSA (artificial)
<400> 8
   gaattacatt caaaaatttt cttctggcat ctgtaatacc g 41
<210> 9
   <211> 40
   <212> DNA
   <213> Artificial Sequence
<220>
   <223> DNA aptamer sequence for heparan sulfate (artificial)
<400> 9
   tgatacaaat actctcaatc aaagccaata tgtcgcaaaa 40
<210> 10
   <211> 40
   <212> DNA
   <213> Artificial Sequence
<220>
   <223> DNA aptamer sequence for heparan sulfate (artificial)
<400> 10
   caaagtaaaa ttaacagata gtacgttctc aatctcgcga 40
<210> 11
   <211> 40
   <212> DNA
   <213> Artificial Sequence
<220>
   <223> DNA aptamer sequence for PIF (artificial)
<400> 11
   tcttcttgca catatttttc tccgtgagac atgtaaataa 40
<210> 12
   <211> 40
   <212> DNA
   <213> Artificial Sequence
<220>
   <223> DNA aptamer sequence for PIF (artificial)
<400> 12
   atgtacttca cttcagtttt ctttaaacac gtttcacata 40
<210> 13
   <211> 39
   <212> DNA
   <213> Artificial Sequence
<220>
   <223> DNA aptamer sequence for PIF (artificial)
<400> 13
   ccgcatttat cagtttaccg ccccataaac ataaccgct 39
<210> 14
   <211> 40
   <212> DNA
   <213> Artificial Sequence
<220>
   <223> DNA aptamer sequence for PIF (artificial)
<400> 14
   aattatgctc atgattttct tcaaaaaggc tcgcgcaatt 40
<210> 15
   <211> 40
   <212> DNA
   <213> Artificial Sequence
<220>
   <223> DNA aptamer sequence for PIF (artificial)
<400> 15
   ggctgttaaa cttacttttc ttcagtaatt gccgttgaca 40
<210> 16
   <211> 40
   <212> DNA
   <213> Artificial Sequence
<220>
   <223> DNA aptamer sequence for PIF (artificial)
<400> 16
   tagtttatca ggagcgatca ctgatcatga gtaactttta 40
<210> 17
   <211> 40
   <212> DNA
   <213> Artificial Sequence
<220>
   <223> DNA aptamer sequence for PIF (artificial)
<400> 17
   atcaagaatt gataatttta ggaattgcgt atcgctgcta 40
<210> 18
   <211> 40
   <212> DNA
   <213> Artificial Sequence
<220>
   <223> DNA aptamer sequence for RNase A (artificial)
<400> 18
   aactgtgttt taggacttca ttgtcttaat tctcttccct 40
<210> 19
   <211> 40
   <212> DNA
   <213> Artificial Sequence
<220>
   <223> DNA aptamer sequence for RNase A (artificial)
<400> 19
   agcgcgatat tacggtctcg aaccaaaacc atcacggttc 40
<210> 20
   <211> 38
   <212> DNA
   <213> Artificial Sequence
<220>
   <223> DNA aptamer sequence for RNase A (artificial)
<400> 20
   atagtttaaa tttaatcttc tgccaccctt cactttca 38
<210> 21
   <211> 40
   <212> DNA
   <213> Artificial Sequence
<220>
   <223> DNA aptamer sequence for laminin (artificial)
<400> 21
   gcacatctcc cgttcgactt ttttatcttc gagcacctaa 40
<210> 22
   <211> 40
   <212> DNA
   <213> Artificial Sequence
<220>
   <223> DNA aptamer sequence for laminin (artificial)
<400> 22
   ccatgtagca caaaacaacg atataagaac tacatttagt 40
<210> 23
   <211> 40
   <212> DNA
   <213> Artificial Sequence
<220>
   <223> DNA aptamer sequence for laminin (artificial)
<400> 23
   ggctttctaa tctaacacga tctcctctcc ttacgccgtg 40
<210> 24
   <211> 40
   <212> DNA
   <213> Artificial Sequence
<220>
   <223> DNA aptamer sequence for laminin (artificial)
<400> 24
   tactcttgtt taaactagaa cagtaaaata ttaattctta 40
<210> 25
   <211> 40
   <212> DNA
   <213> Artificial Sequence
<220>
   <223> DNA aptamer sequence for laminin (artificial)
<400> 25
   tggagtttat aatactcgag gctagtagtg ccattttaca 40
<210> 26
   <211> 40
   <212> DNA
   <213> Artificial Sequence
<220>
   <223> DNA aptamer sequence for laminin (artificial)
<400> 26
   acgcgcggct gtggggaagg tacaggttcc gaacgatgga 40
<210> 27
   <211> 40
   <212> DNA
   <213> Artificial Sequence
<220>
   <223> DNA aptamer sequence for laminin (artificial)
<400> 27
   acaagaacta tttttatcaa agacgtcacc aacttaaggc 40
<210> 28
   <211> 40
   <212> DNA
   <213> Artificial Sequence
<220>
   <223> DNA aptamer sequence for laminin (artificial)
<400> 28
   caaataatcg ttttataatt accaacacat tttggttaac 40
<210> 29
   <211> 40
   <212> DNA
   <213> Artificial Sequence
<220>
   <223> DNA aptamer sequence for laminin (artificial)
<400> 29
   tttatattaa gcaacttttt gagagttgat tgataattta 40
<210> 30
   <211> 40
   <212> DNA
   <213> Artificial Sequence
<220>
   <223> DNA aptamer sequence for IL-7 cat 200-7 (artificial)
<400> 30
   tggcctaatc tcggagactg gccgctgtgg gcgcgggcct 40
<210> 31
   <211> 40
   <212> DNA
   <213> Artificial Sequence
<220>
   <223> DNA aptamer sequence for IL-15 cat 200-15 (artificial)
<400> 31
   tctaattatg ttacaaaata attgttatgc tccgcaaata 40
<210> 32
   <211> 39
   <212> DNA
   <213> Artificial Sequence
<220>
   <223> DNA aptamer sequence for IL-15 cat 200-15 (artificial)
<400> 32
   tcatttaatt gtcctaaatc tgaaaattta ttatatttc 39
<210> 33
   <211> 40
   <212> DNA
   <213> Artificial Sequence
<220>
   <223> DNA aptamer sequence for IL-21 cat 200-21 (artificial)
<400> 33
   ccgggataaa ttactaagtt tcgggtattt tgacaatatt 40
<210> 34
   <211> 40
   <212> DNA
   <213> Artificial Sequence
<220>
   <223> DNA aptamer sequence for IL-21 cat 200-21 (artificial)
<400> 34
   aaacctgcaa aatttagggc caatgtgtgt attgaacggg 40
<210> 35
   <211> 40
   <212> DNA
   <213> Artificial Sequence
<220>
   <223> DNA aptamer sequence for IL-21 cat 200-21 (artificial)
<400> 35
   gttatagtaa tattggttct agctctcagt aatatcaaaa 40
<210> 36
   <211> 40
   <212> DNA
   <213> Artificial Sequence
<220>
   <223> DNA aptamer sequence for IL-21 cat 200-21 (artificial)
<400> 36
   accctataag ctgagataag cattctgtgg acgaaaagtt 40
<210> 37
   <211> 40
   <212> DNA
   <213> Artificial Sequence
<220>
   <223> DNA aptamer sequence for IL-15R (artificial)
<400> 37
   aggcgttaac tcttgtcatg ttatagacgt ctaatccatc 40
<210> 38
   <211> 40
   <212> DNA
   <213> Artificial Sequence
<220>
   <223> DNA aptamer sequence for IL-21R (artificial)
<400> 38
   actgtaatca cttcttttaa atagtcccgg aacgatatca 40
<210> 39
   <211> 40
   <212> DNA
   <213> Artificial Sequence
<220>
   <223> DNA aptamer sequence for IL-21R (artificial)
<400> 39
   cttgatccat actataactt aacatttgtt catctcaagt 40
<210> 40
   <211> 40
   <212> DNA
   <213> Artificial Sequence
<220>
   <223> DNA aptamer for IgG2a (anti CD19) (artificial)
<400> 40
   ctaaatcgcg aaccgagttt ttgtcaaagt tctagattaa 40
<210> 41
   <211> 40
   <212> DNA
   <213> Artificial Sequence
<220>
   <223> DNA aptamer sequence for anti-CD20 (artificial)
<400> 41
   acgaactttt ttaattcgca gacatgttta tagtttcttg 40